# EUROPEAN PATENT SPECIFICATION

(11) **EP 4 308 932 B1**
(45) Date of publication and mention of the grant of the patent: **27.11.2024**
(21) Application number: 22716862.2
(22) Date of filing: 17.03.2022
(51) Int. Cl.: G01N 33/569, A61P 31/14

(54) **METHOD FOR THE DIAGNOSIS OF A CORONAVIRUS INFECTION**
VERFAHREN ZUR DIAGNOSE EINER CORONAVIRUSINFEKTION
PROCÉDÉ DE DIAGNOSTIC D'UNE INFECTION À CORONAVIRUS

(30) Priority: 18.03.2021 EP 21382219
(43) Date of publication of application: 24.01.2024
(73) Proprietor: Gerencia Regional de Salud de Castilla y León, 47007 Valladolid (ES); Universidad de Valladolid, 47002 Valladolid (ES); Fundación Instituto de Estudios Ciencias de la Salud de Castilla y León (IECSCYL-IBSAL), 37007 Salamanca (ES); Instituto de Estudios de Ciencias De La Salud De Castilla Y León (Iecscyl), 42002 Soria (ES)
(72) Inventor: TAMAYO GÓMEZ, Eduardo, 47003 Valladolid (ES); GÓMEZ SÁNCHEZ, Esther, 47003 Valladolid (ES); TAMAYO VELASCO, Álvaro, 47003 Valladolid (ES); MARTÍNEZ DE PAZ, Pedro José, 47005 Valladolid (ES); FERNÁNDEZ MARTÍNEZ, Itziar, 47011 Valladolid (ES); BERNARDO ORDIZ, David, 47003 Valladolid (ES); HEREDIA RODRÍGUEZ, María, 37007 Salamanca (ES); GONZALO BENITO, Hugo, 47003 Valladolid (ES)
(74) Representative: Hoffmann Eitle
(86) International application number: PCT/EP2022/057048
(87) International publication number: WO 2022/195037

(56) References cited:
- US-A1- 2006 040 329
- BÜLOW ANDERBERG SARA ET AL: "Increased levels of plasma cytokines and correlations to organ failure and 30-day mortality in critically ill Covid-19 patients", CYTOKINE, ACADEMIC PRESS LTD, PHILADELPHIA, PA, US, vol. 138, 14 December 2020 (2020-12-14), XP086450118, ISSN: 1043-4666, [retrieved on 20201214], DOI: 10.1016/J.CYTO.2020.155389
- LIU YINGXIA ET AL: "Elevated plasma levels of selective cytokines in COVID-19 patients reflect viral load and lung injury", vol. 7, no. 6, 9 March 2020 (2020-03-09), pages 1003 - 1011, XP055825706, ISSN: 2095-5138, Retrieved from the Internet <URL:http://academic.oup.com/nsr/article-pdf/7/6/1003/38881910/nwaa037.pdf> DOI: 10.1093/nsr/nwaa037
- YOUNG BARNABYE ET AL: "Viral Dynamics and Immune Correlates of Coronavirus Disease 2019 (COVID-19) Severity National Centre for Infectious Diseases", CLINICAL INFECTIOUS DISEASES, 1 January 2020 (2020-01-01), XP055825849, Retrieved from the Internet <URL:https://academic.oup.com/cid/advance-article-pdf/doi/10.1093/cid/ciaa1280/33861329/ciaa1280.pdf> [retrieved on 20210720]
- YOUNG ET AL: "Supplement Appendix: Viral dynamics and immune correlates of COVID- 19 disease severity Contents", 28 August 2020 (2020-08-28), XP055825862, Retrieved from the Internet <URL:https://academic.oup.com/cid/advance-article/doi/10.1093/cid/ciaa1280/5898476#208213649> [retrieved on 20210720]
- JIA RAN ET AL: "Mild Cytokine Elevation, Moderate CD4+ T Cell Response and Abundant Antibody Production in Children with COVID-19", VIROLOGICA SINICA, vol. 35, no. 6, 28 August 2020 (2020-08-28), pages 734 - 743, XP037347496, ISSN: 1674-0769, DOI: 10.1007/S12250-020-00265-8
- HORSPOOL ALEXANDER M ET AL: "Interplay of Antibody and Cytokine Production Reveals CXCL13 as a Potential Novel Biomarker of Lethal SARS-CoV-2 Infection", MSPHERE, vol. 6, no. 1, January 2021 (2021-01-01), pages - 20, XP009536892

## Description

### FIELD OF THE INVENTION

The present invention can be included in the medical field. Particularly, the present invention refers to an *in vitro* method for the diagnosis of a coronavirus infection which is preferably carried out by using minimally-invasive biological samples obtained from the subject, most preferably plasma samples.

### STATE OF THE ART

A new strain of coronavirus, severe acute respiratory syndrome coronavirus 2 (SARS-CoV-2) was recognized in December 2019 in China. Since then, more than 66 million cases have been reported in 184 countries, causing more than 1,500,000 deaths. Incubation time is approximately 5 days. Clinical presentation is broad. Most common symptoms include fever, cough and fatigue. However, it can be also presented from a totally asymptomatic infection to an acute respiratory distress syndrome with multiorgan dysfunction.

Hospitalized patients usually present moderate or severe respiratory disease with high fever and pneumonia. Certain biomarkers have been related to this situation as well as radiological alterations. Nevertheless, clinical, radiological and laboratory features cannot be easily distinguished from pneumonia induced by other viral or bacterial infections.

Precise and early diagnosis is the cornerstone therefore for optimal both prevention and treatment, especially in hospitals. Polymerase chain reaction (PCR) is the main test but its sensitivity is variable and, in some reports, reaches 71%. The correct sample collection is necessary, which requires the performance of new PCR tests if the result is negative and a high probability of infection exist. All of this causes a delay in the diagnosis and optimization of treatment, so necessary not only in each patient survival but also in preventive quarantine and hospital organization.

HORSPOOLALEXANDER M ET AL: "Interplay of Antibody and Cytokine Production Reveals CXCL13 as a Potential Novel Biomarker of Lethal SARS-CoV-2 Infection", MSPHERE, vol. 6, no. 1, January 2021 (2021-01) discloses IP-10 to be elevated compared to healthy controls and that IL-4 levels were significantly decreased in patients who succumbed to infection.

BULOW ANDERBERG SARA ET AL: "Increased levels of plasma cytokines and correlations to organ failure and 30-day mortality in critically ill Covid-19 patients", CYTOKINE, ACADEMIC PRESS LTD, PHILADELPHIA, PA, US, vol. 138, 14 December 2020 (2020-12-14) discloses that high IL-4 levels are often strongly correlated with respiratory failure in Covid-19 patients.

So, there is an unmet medical need of finding new tools for improving the diagnosis of coronavirus infection. The present invention is thus focused on solving this problem and, consequently, a biomarker fingerprint which efficiently contributes to the proper diagnosis of coronavirus infection is herein presented.

### DESCRIPTION OF THE INVENTION

### Brief description of the invention

The present invention refers to an *in vitro* method for the diagnosis of a coronavirus infection which is preferably carried out by using minimally-invasive biological samples obtained from the subject, most preferably plasma samples.

The inventors of the present invention consider that the cytokine profile plays an important role in the pathogenesis of hospitalized patients and, consequently, finding a specific cytokine profile could be of utmost importance for performing an early diagnosis of the coronavirus infection, thus giving rise to the possibility of administering targeted treatments in an early stage of the disease.

Hence, the inventors of the present invention have identified a cytokine fingerprint that allows an early diagnosis of COVID-19. It is also important to note that the present invention is implemented in by using minimally-invasive biological samples obtained from the subject, preferably plasma samples.

Particularly, the examples and figures provided below show strong statistical results with respect to three specific cytokines, particularly: Interferon gamma-induced protein 10 (IP-10), platelet-derived growth factor (PDGFBB) and interleukin 4 (IL4). According to the present invention, any of these cytokines show a relevant statistical result (IP10 shows AUC = 0.92, PDGFBB shows AUC = 0.86 and IL4 shows AUC = 0.67) and may independently contribute to the diagnosis of a coronavirus infection. Moreover, when the three cytokines were combined in a model, an AUC of 0.9557 was obtained with 99.07% of sensitivity and 96.43% of specificity.

So, the present invention describes the molecular fingerprint of cytokines and chemokines that define COVID-19 infection and that therefore could have a clinical impact as novel diagnose biomarkers complementary to the PCR or the antigen tests. Particularly, high levels of IP10 and PDGF-BB coupled with low levels of IL-4 can predict COVID-19 infection. This model was internally validated revealing a 99.07% accuracy. Moreover, another validation in other cohort of patients, the external one, confirmed our results.

In summary, the inventors of the present invention have developed and validated a simple logistic prediction model for COVID-19 disease based on the determination of just 3 blood cytokines: IL4, IP10 and PDGFBB. Their implementation in routine would be easy and quick, associating extraordinary capacities of accuracy, sensitivity and specificity in patients admitted to the hospital. It can be used as a complementary tool to aid clinical decision-making.

So, the first embodiment of the present invention refers to an *in vitro* method for the diagnosis of a coronavirus infection (hereinafter the method of the invention) in a subject which comprises: a) Measuring the level of IP10 in a minimally-invasive biological sample obtained from the subject, and b) wherein if a deviation or variation of the level of IP10 is identified, as compared with a pre-established reference level measured in healthy control subjects, it is indicative that the subject is suffering from coronavirus infection.

In a preferred embodiment, the method of the invention further comprises: a) Measuring the level of PDGFBB or IL4 in a minimally-invasive biological sample obtained from the subject, and b) wherein if a deviation or variation of the level of PDGFBB or IL4 is identified, as compared with a pre-established reference level measured in healthy control subjects, it is indicative that the subject is suffering from coronavirus infection.

In a preferred embodiment, the method of the invention comprises: a) Measuring the level of [IP10, PDGFBB and IL4] in a minimally-invasive biological sample obtained from the subject, and b) wherein if a deviation or variation of the level of [IP10, PDGFBB and IL4] is identified, as compared with a pre-established reference level measured in healthy control subjects, it is indicative that the subject is suffering from coronavirus infection.

In a preferred embodiment, the method of the invention is carried out in in plasma, serum or blood samples obtained from the subject.

In a preferred embodiment, the method of the invention comprises: a) Measuring the level of [IP10 PDGFBB and IL4] in plasma samples obtained from the subject, and b) wherein if a deviation or variation of the level of [IP10 PDGFBB and IL4] is identified, as compared with a pre-established reference level measured in healthy control subjects, this is indicative that the subject is suffering from coronavirus infection.

In a preferred embodiment, if the level of the biomarkers or the risk score value measured in the subject is statistically higher or lower as compared with the level measured in healthy control subjects, this is indicative that the subject is suffering from coronavirus infection. So, according to the method of the invention, after measuring the level of the cytokines, a score value is obtained for the signature and this score value is compared with a threshold value which defines the diagnostic rule. If this score value is higher/lower than the threshold, then the corresponding sample is classified as a positive sample, which is an indication that the patient might be suffering from a coronavirus infection. The threshold value has been defined in order to optimize sensitivity and specificity values. Consequently, in a preferred embodiment, the method of the invention comprises: a) Measuring the level of the above cited combination of cytokines, in a biological sample obtained from the subject, b) processing, using for instance a processor unit, the concentration values in order to obtain a risk score and c) wherein if a deviation or variation of the risk score value obtained for any of the above cited combinations of biomarkers is identified, as compared with a reference value, this is indicative that the subject is suffering from a coronavirus infection.

In this context, a computer comprising a processing unit can be used, which is configured to:
- Receive the level of the cytokines which are measured,
- Process the information received for finding a substantial variation or deviation of the level of the cytokines, and
- Provide an output through a terminal display of the variation or deviation of the cytokine level, wherein said variation or deviation indicates that the subject might be suffering from a coronavirus infection.

In a preferred embodiment, the method of the invention the method of the invention is focused on the diagnosis of a coronavirus infection is caused by SARS-CoV-2.

In a preferred embodiment, the result obtained with the method of the invention is confirmed by an image technique, for example (non-exhaustive list): Echography, CT scan or computed tomography scan (CAT scan) or Magnetic resonance imaging (MRI); or PCR (Polymerase Chain Reaction).

Also disclosed is the in *vitro* use of IP10, [IP10 and PDGFBB], or [IP10, PDGFBB and IL4] for the diagnosis of a coronavirus infection in a subject, departing from a minimally-invasive biological sample obtained from the subject.

In a preferred embodiment, the present invention refers to the use of IP10, [IP10 and PDGFBB], or [IP10, PDGFBB and IL4] for the diagnosis of a coronavirus infection caused by SARS-CoV-2 in a subject departing from a minimally-invasive biological sample obtained from the subject.

In a preferred embodiment, the present invention refers to the use of [IP10, PDGFBB and IL4] for the diagnosis of a coronavirus infection departing from plasma, serum or blood samples obtained from the subject.

Also disclosed is a kit configured for implementing the method of the invention which comprises: a) Reagents for obtaining a plasma sample from the subject, and b) reagents for determining the level of [IP10, PDGFBB and IL4].

Also disclosed is the use of the kit for the diagnosis of a coronavirus infection, preferably a coronavirus infection caused by SARS-CoV-2.

Also disclosed is an antiviral composition for use in the treatment of a coronavirus infection which has been diagnosed by following performing the method of the invention. Alternatively, the present disclosure refers to (not claimed) method for treating patients who may be suffering or are suffering from coronavirus infection and have been diagnosed with the method of the invention, which comprises administering a therapeutically effective amount of an antiviral composition. In a preferred embodiment the antiviral treatment is selected from the treatments disclosed in [Evans, Laura et al., 2021. Surviving Sepsis Campaign: International Guidelines for Management of Sepsis and Septic Shock 2021. Critical Care Medicine: November 2021 - Volume 49 - Issue 11 - p e1063-e1143 doi: 10.1097/CCM. 0000000000005337]*.*

For the purpose of the present invention the following terms are defined:
- The expression "minimally-invasive biological sample" refers to any sample which is taken from the body of the patient without the need of using harmful instruments, other than fine needles used for taking the blood from the patient, and consequently without being harmfully for the patient. Specifically, minimally-invasive biological sample refers in the present invention to: blood, serum, or plasma samples.
- The expression "pre-established reference level" refers to the level measured in healthy control subjects. If a deviation of the level of the cytokines is determined with respect to this "pre-established reference level", it is an indication of coronavirus infection.
- The expression "risk score" refers to a risk value obtained after processing one or more values into a single value (or risk value), which represents the probability of disease for the individual. This risk value will be compared with a reference value to evaluate if the patient might be suffering from coronavirus infection.
- A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtain a classification standard having significance for sample classification. The ROC curve is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis/prognosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve.
- By "comprising" it is meant including, but not limited to, whatever follows the word "comprising". Thus, use of the term "comprising" indicates that the listed elements are required or mandatory, but that other elements are optional and may or may not be present.
- By "consisting of" it is meant "including, and limited to", whatever follows the phrase "consisting of". Thus, the phrase "consisting of" indicates that the listed elements are required or mandatory, and that no other elements may be present.
- By "therapeutically effective dose or amount" is intended an amount that, when administered as described herein, brings about a positive therapeutic response in a subject suffering a coronavirus infection. The exact amount required will vary from subject to subject, depending on the species, age, and general condition of the subject, the severity of the condition being treated, mode of administration, and the like.

### Brief description of the figures

**Figure 1****.** Individual logistic regression models on each cytokine.
**Figure 2****.** Probability of infection when cytokine **A)** IL4, **B)** IP10 or **C)** PDGFBB were measured.

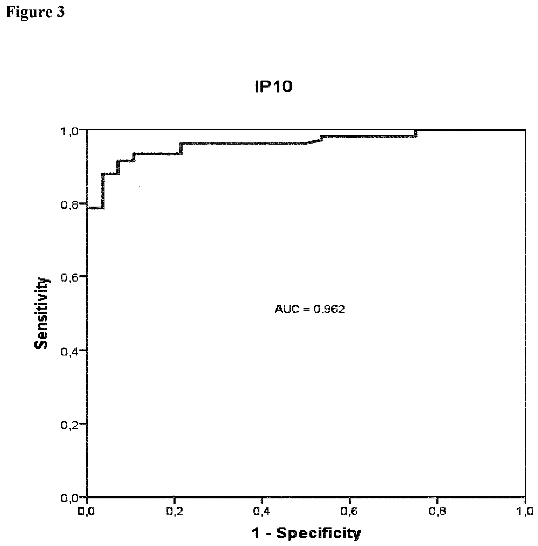

**Figure 3****.** Graph showing IP10 AUC = 0.92.

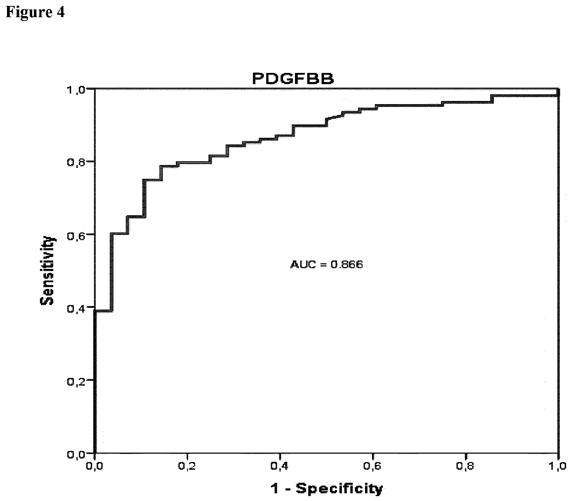

**Figure 4****.** Graph showing PDGFBB AUC = 0.86.

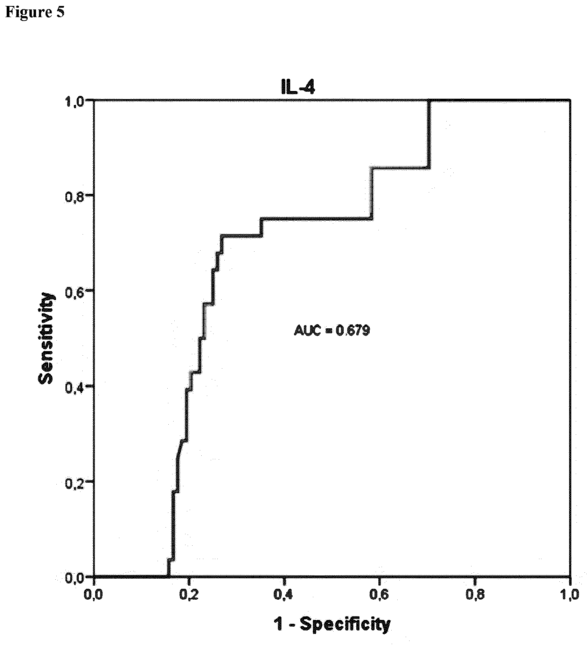

**Figure 5****.** Graph showing IL4 AUC = 0.67.

### Detailed description of the invention

### Example 1. MATERIAL AND METHODS

### Example 1.1. Patient selection

Prospective study including 108 cases and 28 controls. Cases were over 18 years old patients who were diagnosed with COVID-19 and admitted at the Hospital Clinico Universitario (Valladolid, Spain) between the 24^{th} of March and the 11^{th} of April 2020. Positive result in severe acute respiratory syndrome coronavirus 2 (SARS-CoV-2) infection was confirmed by polymerase chain reaction (PCR) testing of a nasopharyngeal sample. Patients with other infections and/or any chronic terminal illnesses were excluded from the study. Controls were represented by 28 age and gender matched healthy volunteers for the normalization of the analytical data of the cytokines. Written consent was obtained by all study participants following ethics approach by the Hospital's Clinical Ethics Committee (PI 20-1717).

### Example 1.2. Biological samples

Blood samples were collected from the patients following their first night at the hospital and 09:00h using 3.2% sodium citrate tubes and centrifuging them at 2000 g for 20 minutes at room temperature. Plasma was aliquoted and cryopreserved at -80°C until used.

### Example 1.3. Cytokines and chemokines analysis

Plasma aliquots were used for the quantification of soluble mediators by the kit 45-plex Human XL Cytokine Luminex Performance Panel (R&D). A duplicate analysis was performed on each sample.

Cytokines or chemokines included in the Panel were BDNF, EGF, Eotaxin (also known as CCL11), FGF-2, GM-CSF, GRO-α (CXCL1), HGF, IFN-α, IFN-γ, IL-1α, IL-1β, IL-10, IL-12p70, IL-13, IL-15, IL-17A (CTLA-18), IL-18, II,-1RA, IL-2, IL-21, IL-22, IL-23, IL-27, IL-31, IL-4, IL-5, IL-6, IL-7, IL-8 (also known as CXCL8), IL-9, IP-1 beta (CCL4), IP-10 (CXCL10), LIF, MCP-1 (CCL2), MIP-1α (CCL3), NGF-β, PDGF-BB, PIGF-1, RANTES (CCL5), SCF, SDF-1α, TNFα, TNFβ, VEGF-A, VEGF-D.

### Example 1.4. Variables

Demographic, clinical and analytical data (Leukocytes, Lymphocytes, Neutrophils, Platelets, C-Reactive Protein, Ferritin and D-Dimer) of each patient were recorded to describe the clinical phenotype.

### Example 1.5. Hospital protocol treatment

The hospital protocol for the treatment of COVID-19 pneumonia in March and April 2020 included: Lopinavir/Ritonavir (Kaletra^{®}. Abbott) 200/50 mg/ml solution twice a day. Hydroxychloroquine (Dolquine^{®}. Rubió) 400 mg twice a day. According to inflammatory criteria, treatment would also add Interferon 1β (Betaferon^{®}. Bayer) 0.25 mg every 48 hours, corticosteroids 240 mg every day for three days, Tocilizumab (RoActemra ^{®}. Roche), Baricitinib (Olumiant^{®}. Lilly) or Anakinra (Kineret^{®}. Amgen). In case of suspected bacterial superinfection, antibiotic treatment was required. Oxygen support (nasal cannula, high flow nasal cannula and non-invasive or invasive mechanical ventilation) was administered to patients based on the severity of hypoxemia.

### Example 1.6. Statistical analysis

Demographic and clinical characteristics were evaluated using the Chi-square test for categorical variables and the Mann-Whitney U test for continuous variables when appropriate. Mean values and 95% confidence intervals (95% CI) were used to describe quantitative variables, while median values and interquartile range (IR) were used for ordinal ones.

Individual logistic regression models, in which the exponential of the coefficients can be directly interpreted as odds ratio, was applied. Multivariate model used all those markers that showed a certain degree of association with the disease. A cutoff point < 0.1 in the p-value was required in the individual model. When there are a large number of variables to be included in the multivariate model, especially when the sample size is small, the problem arises of selecting a good subset of variables that intervene in the multivariate model. To overcome that, we used an algorithm that lists all possible sets of biomarkers, leaving us with the best model. The choice of the model was made using the akaike information criterion (AIC).

Internal validation tried to determine the discrimination capacity of the model and its capacity to classify new individuals correctly. As a global measure, we use the area under the ROC curve (AUC), knowing that a model is a perfect classifier when the AUC is 1.

For cytokine analysis, to impute cytokine values below detection limit, robust regression on order statistics was used: this method performs a regression to impute low values assuming log-normal quantiles for samples with a detection rate of at least 30%, after checking that the data follows a log-normal distribution. To accomplish this, the non-detects and data analysis (NADA) R package was used. Molecules detected in less than 30% of the samples were not statistically analyzed any further. Cytokine expression data were transformed using the logarithmic base 2 scale. Pro-inflammatory/anti-inflammatory cytokine ratios were also calculated.

Statistical analysis was performed by a PhD-licensed statistician (co- author IF) using the R statistical package version 3.1.1 (R Core Team; Foundation for Statistical Computing, Vienna, Austria; URL: https:// www.R.-project.org/). Statistical significance was set at *P* ≤ 0.05.

### Example 2. RESULTS

### Example 2.1. Statistical results

There were no statistical differences in gender and age among cases and controls (**Table 1**). Median age was 69 years in the COVID-19 group, with 55.3% male, while the control group median age was 71 years, with male the 57.1%. Main comorbidities in the cases were hypertension (46.3%), diabetes (17.6%), chronic obstructive pulmonary disease (16.8%) and coronary disease (13%). Referred to laboratory variables, there were statistically significant differences between both groups as COVID patients had lower leukocytes, lymphocyte and platelets and higher neutrophils levels. Patients were hospitalized for a median of 12 days, which was increased up to 19 days in those who required access to the intensive care units. Finally, death rate of the COVID-19 patients was 22.22% (24 patients in total).

**Table 1**

| | **Cases (N = 108)** | **Control (N = 28)** | **P value** |
|---|---|---|---|
| Age [years. median (IQR)] | 69 (20) | 71 (37) | |
| Male [%(n)] | 55.3% (73) | 57.1% (16) | NS |

| **-Comorbidities, [%(n)]** | | | |
|---|---|---|---|
| Use of tobacco | 8.3% (9) | 10,7% (3) | NS |
| Use of alcohol | 2.8% (3) | 7,14% (2) | NS |
| Heart disease | 13. 0% (14) | 21,42% (6) | NS |
| Diabetes | 17.6% (19) | 14,28% (4) | NS |
| Stroke | 2.8% (3) | 3,57% (1) | NS |
| Hypertension | 46.3% (50) | 50% (14) | NS |
| Liver disease | 1.9% (2) | 0% (0) | NS |
| Obesity | 9.3% (10) | 3,57% (1) | NS |
| COPD | 16.8% (18) | 10,7% (3) | NS |
| Kidney disease | 2.8% (3) | 0% (0) | NS |

| **-Laboratory, [median (IQR)]** | | | |
|---|---|---|---|
| White blood cell count (n°/ml) | 6680 (3135) | 6870 (2155) | <0.001 |
| Lymphocyte count (n°/ml) | 1090 (905) | 2255 (940) | <0.001 |
| Neutrophil count (n°/ml) | 4465 (3225) | 3760 (1340) | <0.001 |
| Platelet count (cell/mm3) | 218500 (108500) | 250000 (58000) | 0.005 |
| D-dimer (ng/mL) | 758 (1230) | | |
| C-reactive protein (mg/L) | 81 (112) | | |

| **-Hospital meters, [median (IQR)]** | | | |
|---|---|---|---|
| Length of hospital stay | 12(13) | | |
| Length of ICU stay | 19(15) | | |

| **-Mortality, [%(n)]** | | | |
|---|---|---|---|
| Exitus 28 days | 18.5% (20) | | |
| Exitus | 22.2% (24) | | |

### Clinical characteristics. Continuous variables are represented as [median, (interquartile range, IQR)]; categorical variables are represented as [%, (n)]. COPD, chronic obstructive pulmonary disease.

Of the total 45 studied cytokines (see **Table 2** showing the level of cytokine comparing cases and control) , 8 out of them had to be excluded (FGF-2, IL-12, IL-21, IL-23, IL-31, IL-9, NGFβ and TNFβ) as they could not be detected in at least 70% of the patients. Of the remaining 37 cytokines, 24 showed statistically significant differences between the patients and the controls (**Table 2**). Most of these cytokines were increased in the patients (BNDF, HGF, IL-1α IL-1β, IL-15, IL-17A, IL-18, IL-1RA, IL-2, IL-6, IL-7, IP1b, IP10, MCP1, PDGF-BB, RANTES, VEGFA and VEGFB). Of note, IL-1RA and PDFGBB were over 10 times expanded in the patients referred to the control group. On the other hand, a total of 6 cytokines (Eotaxin, IFNγ, IL-22, IL-27, IL-4 and SDF1α) were decreased in the COVID-19 patients.

**Table 2**

| **Cytokine** | **Control N = 28** | | **Cases N = 108** | | **P value** |
|---|---|---|---|---|---|
| | **Median** | **IQR** | **Median** | **IQR** | |
| BDNF | 39.01 | 115.95 | 58.67 | 132.52 | **0.024** |
| EGF | 3.78 | 1.48 | 2.15 | 7.4 | 0.171 |
| Eotaxin | 23.82 | 12.66 | 13.85 | 9.6 | **<0.001** |
| GMCSF | 15.07 | 6.19 | 11.38 | 25.39 | 0.733 |
| GROα | 4.26 | 1.83 | 2.99 | 4.17 | 0.190 |
| HGF | 71.45 | 53.6 | 163.75 | 251.66 | **<0.001** |
| IFNα | 0.74 | 0.37 | 0.48 | 1.63 | 0.499 |
| IFNg | 12.56 | 3.4 | 8.81 | 6.7 | **0.005** |
| IL-1α | 0.58 | 0.56 | 2.5 | 7.76 | **0.012** |
| IL-1β | 2.49 | 0.96 | 6.62 | 10.47 | **<0.001** |
| IL-10 | 1.81 | 0.5 | 1.69 | 2.54 | 0.611 |
| IL-13 | 3.17 | 1.5 | 2.06 | 2.86 | 0.093 |
| IL-15 | 4.41 | 2.96 | 13.32 | 17.99 | **<0.001** |
| IL-17A | 2.03 | 0.61 | 7.03 | 14.73 | **<0.001** |
| IL-18 | 14.53 | 18.43 | 47.2 | 51.74 | **<0.001** |
| IL-1RA | 72.51 | 38.73 | 604.75 | 1007.92 | **<0.001** |
| IL-2 | 7.59 | 1.61 | 13.39 | 19.64 | **<0.001** |
| IL-22 | 18.15 | 22.33 | 3.57 | 20.89 | **0.015** |
| IL-27 | 38.87 | 18.2 | 18.06 | 34.08 | **0.023** |
| IL-4 | 10.37 | 4.52 | 5.59 | 6.98 | **0.04** |
| IL-5 | 9.42 | 4.88 | 5.12 | 17.34 | 0.394 |
| IL-6 | 8.69 | 3.56 | 13.07 | 22.73 | **0.01** |
| IL-7 | 0.72 | 0.31 | 1.67 | 2.88 | **<0.001** |
| IL-8 | 3.25 | 1.69 | 2 | 3.81 | 0.079 |
| IP1b | 29.95 | 22.42 | 48.6 | 44.16 | **0.007** |
| IP10 | 5.56 | 4.2 | 45.67 | 40.46 | **<0.001** |
| LIF | 12.18 | 2.46 | 14.9 | 16.81 | 0.105 |
| MCP1 | 16.78 | 14.57 | 37.88 | 31.55 | **<0.001** |
| MIP1a | 2.91 | 1.12 | 3.39 | 11.38 | 0.607 |
| PDGF-BB | 31.8 | 39.64 | 303.42 | 656.93 | **<0.001** |
| PIGF1 | 5.82 | 5.16 | 5.45 | 60.52 | 0.807 |
| RANTES | 17.62 | 8.36 | 22.77 | 18.9 | **0.001** |
| SCF | 9.52 | 12.13 | 6.51 | 7.49 | 0.199 |
| SDF1a | 951.75 | 412.62 | 669.5 | 607.62 | **0.011** |
| TNFα | 6.46 | 3.29 | 6.02 | 10.91 | 0.541 |
| VEGFA | 47.8 | 98.36 | 122.65 | 188.12 | **0.001** |
| VEGFD | 6.55 | 10.74 | 12.65 | 13.28 | **0.005** |

| | | | | | |
|---|---|---|---|---|---|
| *Level of cytokine comparing cases and control* | | | | | |

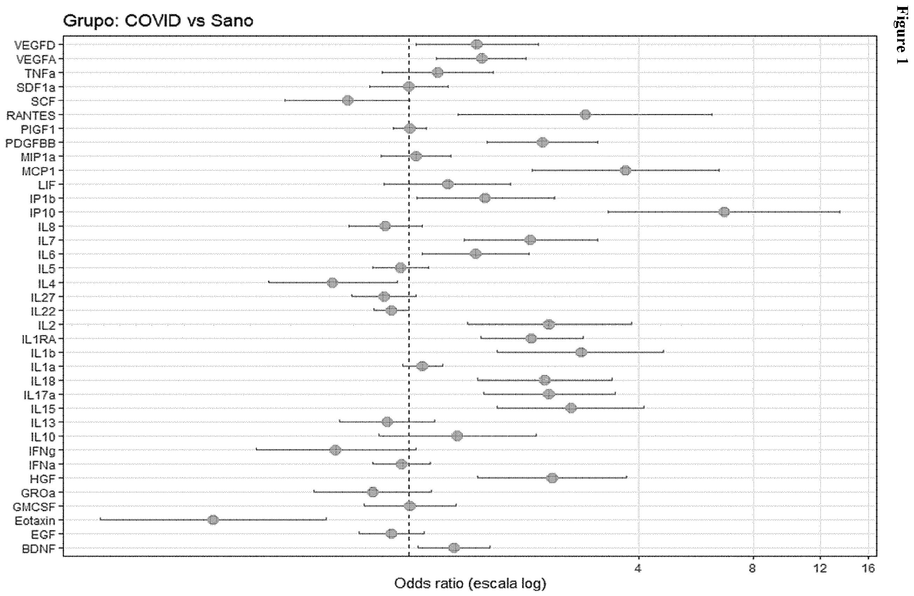

The individual logistic regression models, adjusting the results for age and gender, showed statistically significant overexpression of several cytokines (BDNF, HGF, IL-1β, IL-15, IL-17A, IL-18, IL-1RA, IL-2, IL-6, IL-7, IP1b, IP10, MCP1, PDGFBB, VEGFA, VEGFD, RANTES) while IFN γ, IL-22, IL-4 and SCF were under-expressed (**Figure 1**).

The best multivariate model, the one with the lower AIC, was chosen. After M3 it was not possible to find valid models. In our case, we chose model 3 (AIC =25.12) that included 3 cytokines: IL-4, IP10 and PDGF-BB. See **Table 3** wherein it is identified the best multivariate model following AIC ("Akaike's Information Criterion").

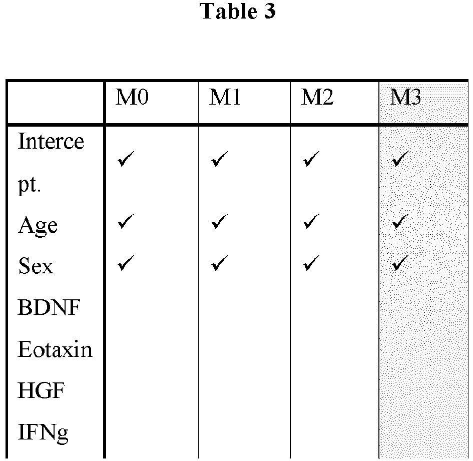

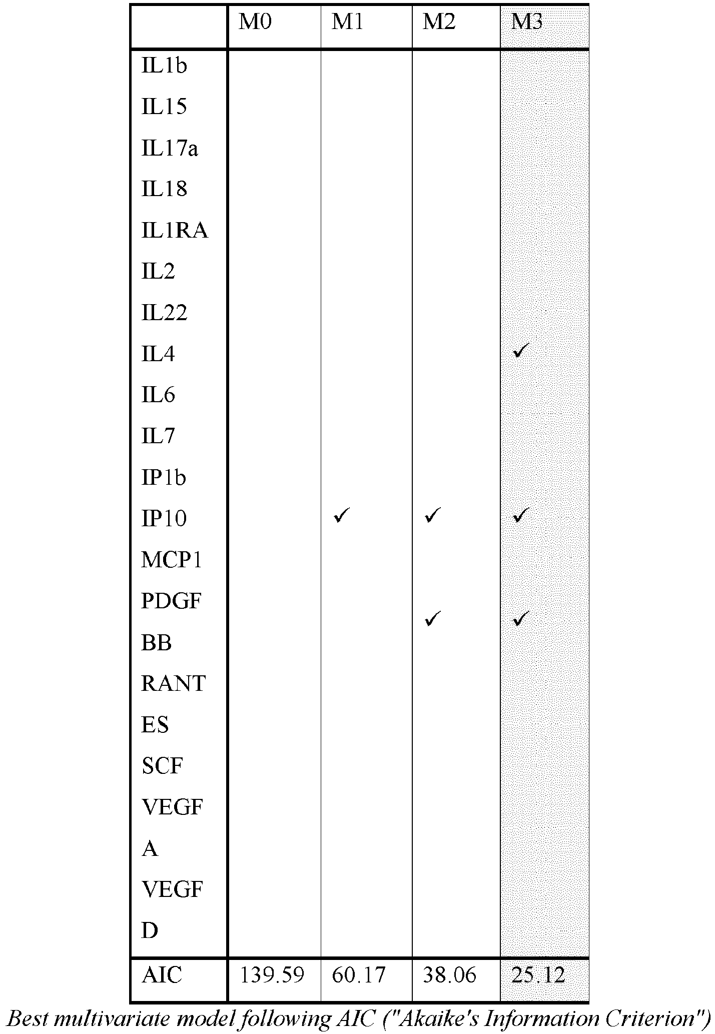

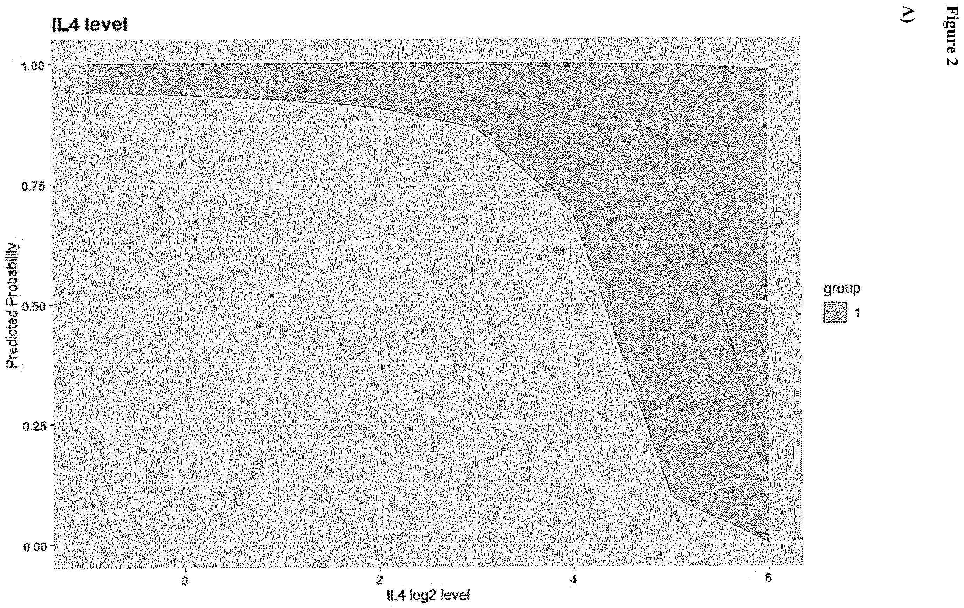

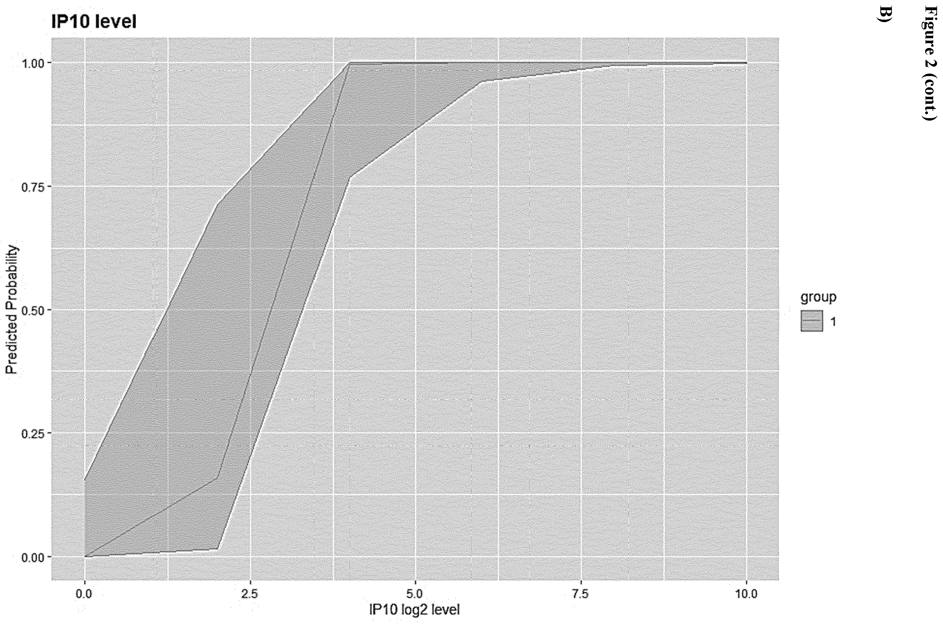

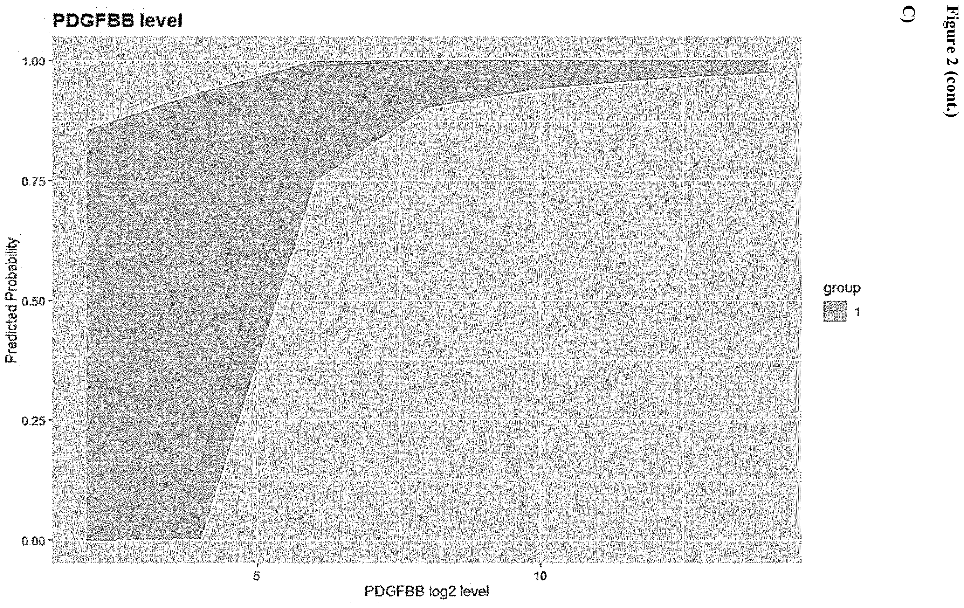

This multivariate model showed a clear association between the levels of these 3 cytokines and the high probability of COVID-19 infection (see **Table 4** showing the multivariate model and the association between cytokine levels and risk of Covid infection. CI, confidence interval; OR, Odds ratio). High IP10 [OR 55.53, 95% CI (2.49-1027.43), p = 0.011] and PDGF-BB [OR 22.49, 95% CI (1.22-412.76), p = 0.036] levels increased the risk of infection, reaching 50 and 22 times more risk if the values were doubled. Low IL-4 [OR 0.04, 95% CI (0.016-0.973), p = 0.048] levels also increased the risk due to their under expression in the patients. According to the level of each cytokine, Figure 2 represents the probability of infection.

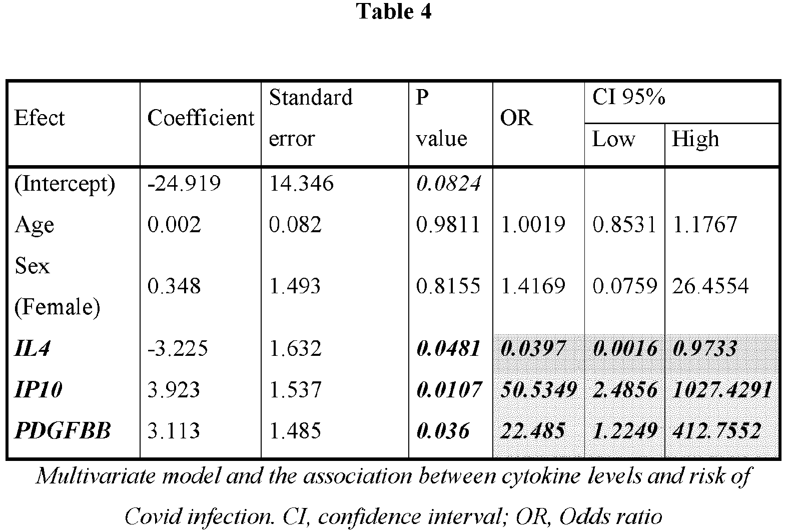

Internal validation showed an AUC of **0.9557. Table 5** shows internal validation using the AUC (Area Under the Curve) and CI (Confident Interval). Hence, our model is especially sensitive to identify COVID-19 patients, with a 99.07% of sensitivity, 96.43 % of specificity and 98.53% of accuracy.

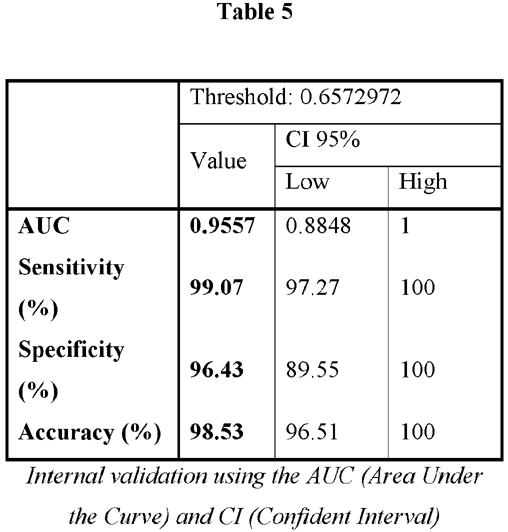

## Claims

1. *In vitro* method for the diagnosis of coronavirus infection in hospitalized patients which comprises: a) Measuring the level of IP10, PDGFBB and IL4 in a minimally-invasive biological sample obtained from the hospitalized patient, and b) wherein the identification of higher levels of IP10 and PDGFBB coupled with low levels of IL-4, as compared with a pre-established reference level measured in healthy control subjects, is indicative that the hospitalized patient is suffering from coronavirus infection.

2. *In vitro* method for the diagnosis of coronavirus infection in hospitalized patients, according to claim 1, which comprises: a) Measuring the level of a group of cytokines consisting of: IP10, PDGFBB and IL4 in a minimally-invasive biological sample obtained from the hospitalized patient, and b) wherein the identification of higher levels of IP10 and PDGFBB coupled with low levels of IL-4, as compared with a pre-established reference level measured in healthy control subjects, is indicative that the hospitalized patient is suffering from coronavirus infection.

3. *In vitro* method for the diagnosis of coronavirus infection in hospitalized patients, according to any of the previous claims, in plasma, serum or blood samples obtained from the hospitalized patient.

4. *In vitro* method for the diagnosis of coronavirus infection in hospitalized patients, according to any of the previous claims, which comprises: a) Measuring the level of a group of cytokine consisting of: IP10, PDGFBB and IL4 in plasma samples obtained from the hospitalized patient, and b) wherein the identification of higher levels of IP10 and PDGFBB coupled with low levels of IL-4, as compared with a pre-established reference level measured in healthy control subjects, this is indicative that the hospitalized patient is suffering from coronavirus infection.

5. *In vitro* method, according to any of the previous claims, for the diagnosis of a coronavirus infection caused by SARS-CoV-2.

6. *In vitro* method, according to any of the previous claims, wherein the diagnosis is confirmed by an image technique or PCR.

## Patentansprüche

1. *In-vitro*-Verfahren zur Diagnose einer Coronavirusinfektion in hospitalisierten Patienten, welches Folgendes umfasst: a) das Messen des Spiegels von IP10, PDGFBB und IL4 in einer minimal-invasiven biologischen Probe erhalten aus dem hospitalisierten Patienten, und b) wobei die Identifizierung von höheren Spiegeln von IP10 und PDGFBB verbunden mit niedrigen Spiegeln von IL-4, im Vergleich zu einem vorab festgesetzten Referenzspiegel gemessen in gesunden Kontrollindividuen, darauf schließen lässt, dass der hospitalisierte Patient unter einer Coronavirusinfektion leidet.

2. *In-vitro*-Verfahren zur Diagnose einer Coronavirusinfektion in hospitalisierten Patienten nach Anspruch 1, welches Folgendes umfasst: a) das Messen des Spiegels von einer Gruppe von Zytokinen bestehend aus: IP10, PDGFBB und IL4 in einer minimal-invasiven biologischen Probe erhalten aus dem hospitalisierten Patienten, und b) wobei die Identifizierung von höheren Spiegeln von IP10 und PDGFBB verbunden mit niedrigen Spiegeln von IL-4, im Vergleich zu einem vorab festgesetzten Referenzspiegel gemessen in gesunden Kontrollindividuen, darauf schließen lässt, dass der hospitalisierte Patient unter einer Coronavirusinfektion leidet.

3. *In-vitro*-Verfahren zur Diagnose einer Coronavirusinfektion in hospitalisierten Patienten nach einem der vorhergehenden Ansprüche, in Plasma-, Serum- oder Blutproben erhalten aus dem hospitalisierten Patienten.

4. *In-vitro*-Verfahren zur Diagnose einer Coronavirusinfektion in hospitalisierten Patienten nach einem der vorhergehenden Ansprüche, welches Folgendes umfasst: a) das Messen des Spiegels von einer Gruppe von Zytokinen bestehend aus: IP10, PDGFBB und IL4 in Plasmaproben erhalten aus dem hospitalisierten Patienten, und b) wobei die Identifizierung von höheren Spiegeln von IP10 und PDGFBB verbunden mit niedrigen Spiegeln von IL-4, im Vergleich zu einem vorab festgesetzten Referenzspiegel gemessen in gesunden Kontrollindividuen, darauf schließen lässt, dass der hospitalisierte Patient unter einer Coronavirusinfektion leidet.

5. *In-vitro*-Verfahren nach einem der vorhergehenden Ansprüche, zur Diagnose einer Coronavirusinfektion verursacht durch SARS-CoV-2.

6. *In-vitro*-Verfahren nach einem der vorhergehenden Ansprüche, wobei die Diagnose mittels einer Bildtechnik oder PCR bestätigt wird.

## Revendications

1. Procédé *in vitro* de diagnostic d'une infection à coronavirus chez des patients hospitalisés qui comprend : a) mesurer le niveau d'IP10, de PDGFBB et d'IL4 dans un échantillon biologique mini-invasif obtenu du patient hospitalisé, et b) dans lequel l'identification de niveaux plus élevés d'IP10 et de PDGFBB couplés avec des niveaux réduits d'IL-4, par rapport à un niveau de référence préétabli mesuré chez des sujets de contrôle sains, indique que le patient hospitalisé souffre d'une infection à coronavirus.

2. Procédé *in vitro* de diagnostic d'une infection à coronavirus chez des patients hospitalisés, selon la revendication 1, qui comprend : a) mesurer le niveau d'un groupe de cytokines consistant en : IP10, PDGFBB et IL4 dans un échantillon biologique mini-invasif obtenu du patient hospitalisé, et b) dans lequel l'identification de niveaux plus élevés d'IP10 et de PDGFBB couplés avec des niveaux réduits d'IL-4, par rapport à un niveau de référence préétabli mesuré chez des sujets de contrôle sains, indique que le patient hospitalisé souffre d'une infection à coronavirus.

3. Procédé *in vitro* de diagnostic d'une infection à coronavirus chez des patients hospitalisés, selon l'une quelconque des revendications précédentes, dans des échantillons de plasma, sérum ou sang obtenus du patient hospitalisé.

4. Procédé *in vitro* de diagnostic d'une infection à coronavirus chez des patients hospitalisés, selon l'une quelconque des revendications précédentes, qui comprend : a) mesurer le niveau d'un groupe de cytokines consistant en : IP10, PDGFBB et IL4 dans des échantillons de plasma obtenus du patient hospitalisé, et b) dans lequel l'identification de niveaux plus élevés d'IP10 et de PDGFBB couplés avec des niveaux réduits d'IL-4, par rapport à un niveau de référence préétabli mesuré chez des sujets de contrôle sains, indique que le patient hospitalisé souffre d'une infection à coronavirus.

5. Procédé *in vitro,* selon l'une quelconque des revendications précédentes, pour le diagnostic d'une infection à coronavirus causée par SARS-CoV-2.

6. Procédé *in vitro,* selon l'une quelconque des revendications précédentes, dans lequel le diagnostic est confirmé par une technique d'imagerie ou par PCR.
